(19) Europäisches Patentamt
European Patent Office
Office européen des brevets

(11) Publication number : **0 454 385 A1**

# (12) EUROPEAN PATENT APPLICATION

(21) Application number : **91303564.8**

(51) Int. Cl.$^5$ : **C07D 473/38**, C07D 473/00

(22) Date of filing : **22.04.91**

(30) Priority : **23.04.90 US 512703**

(43) Date of publication of application :
**30.10.91 Bulletin 91/44**

(84) Designated Contracting States :
**AT BE CH DE DK ES FR GB GR IT LI LU NL SE**

(71) Applicant : **ORTHO PHARMACEUTICAL CORPORATION**
**U.S. Route 202 P.O. Box 300**
**Raritan New Jersey 08869-0602 (US)**

(72) Inventor : **Barton, Donald L.**
**RD 1, Box 478**
**Upper Black Eddy, PA 18972 (US)**
Inventor : **Hajos, Zoltan G.**
**36 Bainbridge Street**
**Princeton, NJ 08540 (US)**
Inventor : **Press, Jeffrey B.**
**716 Buckley Circle**
**Penllyn, PA 19422 (US)**
Inventor : **Sawyers, Rebecca A.**
**717 Landis Avenue**
**Vineland, NJ 08360 (US)**

(74) Representative : **Fisher, Adrian John et al**
**CARPMAELS & RANSFORD 43 Bloomsbury Square**
**London WC1A 2RA (GB)**

## (54) Process for preparing optically active isomers of 6-substituted purinyl piperazine derivatives.

(57) A process for synthesizing optically active isomers of 6-substituted purinyl piperazine derivatives is described. The optically active isomers prepared by the process are useful as cardiotonic agents and antiarrhythmic agents.

EP 0 454 385 A1

## BACKGROUND OF THE INVENTION

### Field of the Invention

The present invention relates to a process for preparing optically active isomers of compounds of the formula:

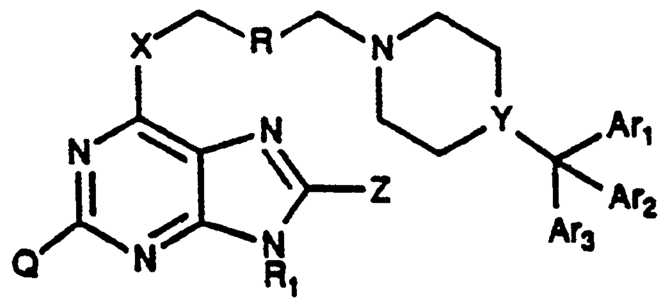

as further described herein. The optically active isomers prepared by the process have positive inotropic activity and are especially useful as cardiotonic agents for improving cardiac ejection in acute and chronic heart failure. The optically active isomers are also useful as antiarrhythmic agents for the treatment or prevention of cardiac arrhythmia. One of these optical isomers was found to contain the desired pharmaceutical properties.

### Description of the Prior Art

Many chemical substances in nature are optically active, i.e. they are either right-handed or left-handed mirror images of each other. Many of these substances play important roles in biological processes, e.g. carbohydrates, amino acids, peptides, proteins, enzymes, alkaloids, etc. This stereospecificity of the optically active systems plays an important role in the development of compounds of biological activity, e.g. Stereochemistry and Biological Activity of Drugs, E.J. Ariens ed. (Blackwell, 1983).

A known process for preparing racemic mixtures of 6-substituted purinyl piperazine derivatives is described in United States Patent No. 4,876,257, and shown below.

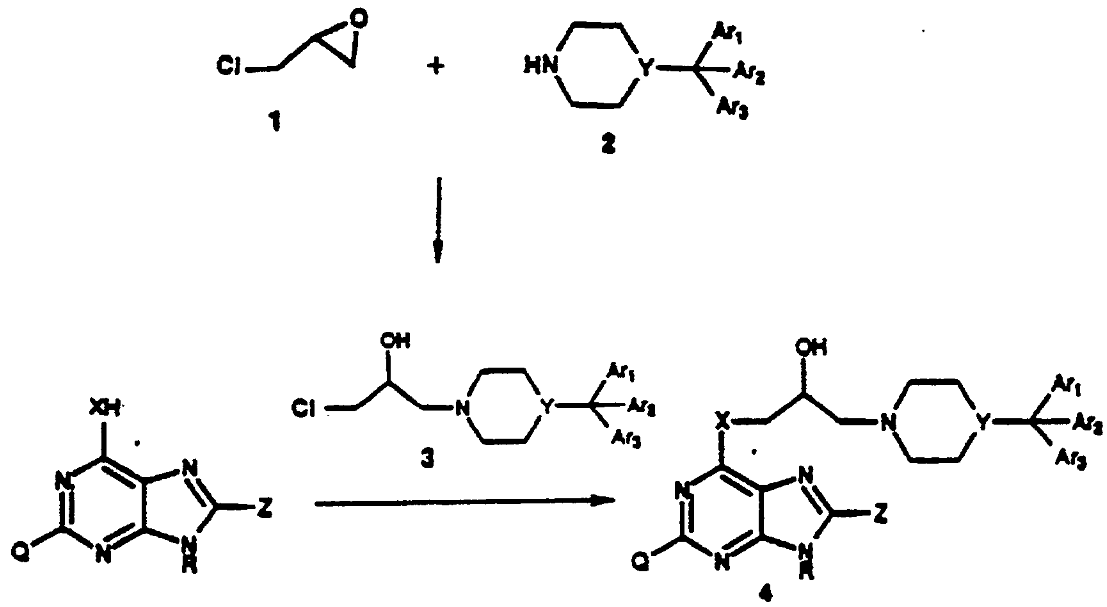

1

2

3

4

This process comprises the addition of epichloro-hydrin (1) with a substituted piperazine (2) to produce a product (3) which is subsequently condensed with a purine derivative to produce the racemic end product (4), as shown below.

There are several ways to achieve optical activity in the synthesis of compounds of pharmacological interest. Chemical separation of the optical isomers results in optical resolution. However, this procedure has the major disadvantage of providing theoretically no more than half of the desired optical isomer based on racemic starting material.

An alternate procedure is asymmetric transformation which can be achieved either microbiologically (enzymes) or chemically (using asymmetric template molecules). A further option is that optically active compounds can be prepared by utilizing chiral building blocks (chiral synthons) in a highly stereospecific synthetic process.

The current art (Sharpless et al., J. Org. Chem. 54, 1295 (1989)) also teaches that some nucleophiles such as phenols and organometallic reagents catalyzed by copper halides will react with either (2R)-(-)-glycidyl tosylate or (2R)-(-)-glycidyl 3-nitrobenzenesulfonate and (2S)-(+)-glycidyl tosylate or (2S)-(+)-glycidyl 3-benzenesulfonate under carefully controlled conditions to give products with high enantiomeric excesses (ee) of optically pure materials. Sharpless et al. also teach that the use of the sodium salts of phenols in the solvent dimethylformamide (DMF) give highly optically pure products.

## SUMMARY OF THE INVENTION

The present invention is directed to a process for preparing optically active isomers of 6-substituted purinyl piperazine derivatives of the general formula:

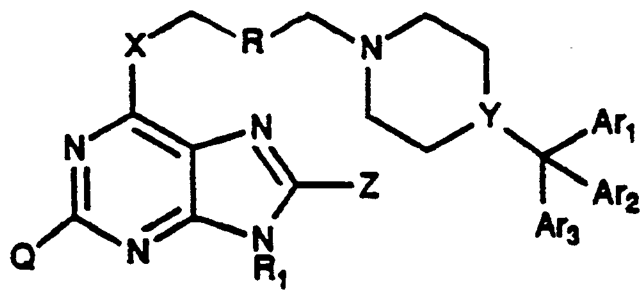

wherein

R may be

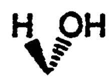 or 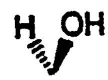 ;

$R_1$ may be hydrogen, $C_1$-$C_4$ alkyl, cycloalkyl, benzyl, $C_1$-$C_4$ alkenyl, $C_1$-$C_4$ alkynyl, epoxypropyl or cyclic ether;

X may be S, O, NH or $NR_2$ wherein $R_2$ may be $C_1$-$C_4$ alkyl;

Y may be N or C=;

$Ar_1$, $Ar_2$ and $Ar_3$ may be independently hydrogen, $C_1$-$C_4$ alkyl, phenyl, substituted phenyl wherein the substituent is $C_1$-$C_4$ alkyl, $C_1$-$C_4$ alkoxy, trifluoromethyl, halo such as fluoro, chloro, bromo and iodo or perhalo, naphthyl, pyridyl or thienyl, wherein at least one of $Ar_1$, $Ar_2$ or $Ar_3$ is aromatic;

Z may be hydrogen, CN or $CO_2R_3$ wherein $R_3$ may be hydrogen, $C_1$-$C_4$ alkyl, hydroxy or halogen such as fluoro, chloro or bromo; and

Q may be hydrogen, halogen, amino, $C_1$-$C_4$ alkyl or hydroxy; with the proviso that when Y is C=, $Ar_3$ is not present.

The process comprises the steps of:

a) reacting an optically active glycidyl-derivative of the formula

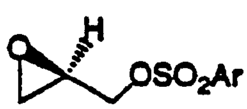 or 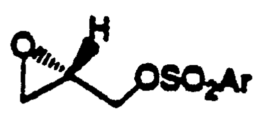

wherein Ar is a 4-methylphenyl group or a 3-nitrophenyl group with a piperazine derivative salt of the formula

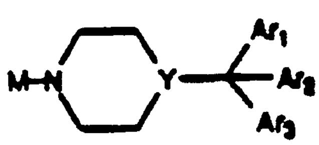

wherein M is Na, K or Li, in a solvent to produce an optically active epoxypiperazine derivative of the formula

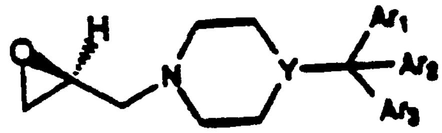

;

and

b) reacting the optically active epoxypiperazine derivative with a sodium salt of a purine derivative of the formula

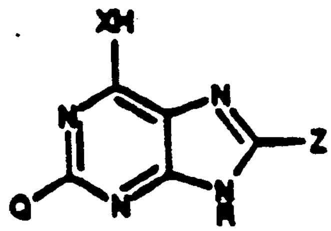

The optically active isomers prepared by this process are useful as cardiovascular agents, and in particular as cardiotonic agents. The isomers are also useful as antiarrhythmic agents.

DETAILED DESCRIPTION OF THE INVENTION

The invention in its broadest aspects relates to a process for preparing optically active isomers of 6-substituted purinyl piperazine derivatives. These optically active isomers exhibit positive inotropic activity.

The process for preparing one of the optically active isomers is outlined below in Scheme 1.

SCHEME 1

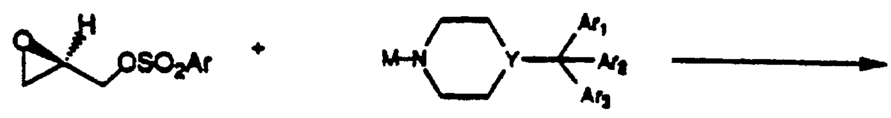

(2R)-(-)-6 a. Ar = 4-Me-Ph
6 b. Ar = 3-$NO_2$-Ph

2-M salt

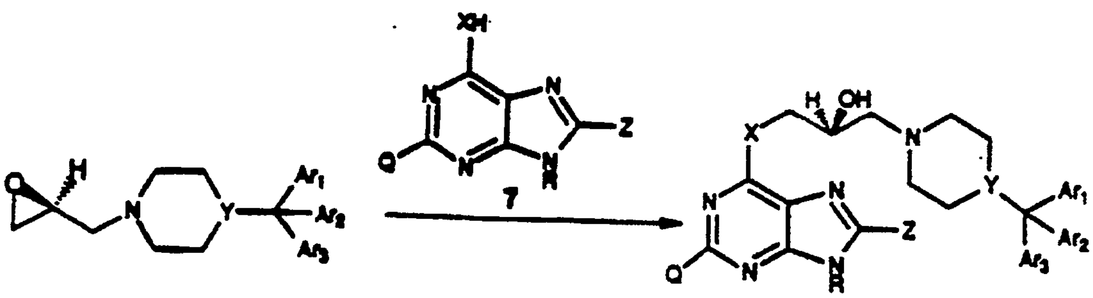

7

(2S)-(-)-5

(2S)-(+)-4

In this process, an optically active glycidyl derivative ((2R)-(-)-6a= glycidyl tosylate; or (2R)-(-)-6b = glycidyl 3-nitrobenzenesulfonate) is reacted with a salt of a piperazine derivative (2-M salt) in a solvent to give an optically active epoxypiperazine derivative ((2S)-(-)-5). The piperazine derivative salts may be Na or K and useful solvents include tetrahydrofuran (THF) and dimethylformamide (DMF). The optically active epoxyiperazine derivative ((2S)-(-)-5) is then reacted with the sodium salt of a purine derivative (7) to yield an optically active 6-substituted purinyl piperazine derivative ((2S)-(+)-4).

The final product is generally present in a ratio of about an 83:17 mixture of the (2S):(2R) enantiomers. This ratio is determined by high pressure liquid chromatography (HPLC) using a chiral column packing for the separation of the pivaloate derivatives.

Since the addition of (2S)-(-)-5 to the sodium salt of the purine derivative (7) does not occur at a chiral site, the ratio of (2S)-(+)-4:(2R)-(-)-4 is a direct consequence of the degree of the regioselectivity of the condensation of (2R)-(-)-6a or (2R)-(-)-6b with 2-M salt. Thus, there are also concomitant lower enantiomeric excesses (ee) of the optically active intermediate ((2S)-(-)-5) prior to formation of the final product ((2S)-(+)-4).

In an alternative process, the starting materials (chiral synthons) for (2S)-(+)-4 may be (2S)-(+)-6a = glycidyl tosylate or (2S)-(+)-6b = glycidyl 3-nitrobenzenesulfonate. When these chiral synthons are used as the starting material the Li salt of 2-M is used to produce the intermediate optically active epoxypiperazine derivative and ultimately the optically active final product.

Alternatively, the opposite enantiomeric final product (2R)-(-)-4 can be prepared by using either (2S)-(+)-6a in the first process or (2R)-(-)-6a in the alternative process described above.

The chiral synthons ((2R)-(-)-6a, (2R)-(-)-6b, (2S)-(+)-6a and (2S)-(+)-6b) are commercially available from the Aldrich Chemical Company, and their synthesis has been described by Sharpless et al., in J. Org. Chem. 51, 3710 (1986) and in J. Org. Chem. 54, 1295 (1989).

Certain characteristics and differences in yields of the final product of this process are noted when different combinations of starting materials and solvents are used. These characteristics and differences in final product yield are set forth in Tables 1 and 2, as well as Figure 1 and the discussion below.

In order to compare the differences in final product yields, the piperazine derivative used for the 2-M salt was always 4-(bis(4-fluorophenyl)methyl) piperazine, and the purine derivative (7) was always 6-mercaptopurine. In this way, chiral synthons could be varied, the 2-M salt could be varied between Na, K and Li, and the solvent could be varied between THF and DMF, yet the final product yield to be determined would always be for the same compounds, i.e. (2S)-(+)-or (2R)-(-)-6-[1-(1-(bis(4-fluorophenyl)methyl)piperazin-4-yl)-2-hydroxy-3-propanylthio]purine.

An unexpected and dramatic difference occurred when the (2R)-(-)-6a starting material and the potassium salt 2-M (M = K) was used in DMF; the desired product ((2S)-(+)-4) was obtained in much higher optical purity. The amount of the (2S) enantiomer was 97.2% by the above mentioned analytical method. (See Batch D, in Table 1, below).

When the sodium (M = Na) or potassium (M = K) salt 2-M is used, the glycidyl compound (2R)-(-)-6 is attacked at C-1 as described below (Figure 1). In contrast, the lithium salt 2-Li attacks at C-3 resulting in the opposite enantiomer of the optically active system (Figure 1; Tables 1 and 2). Thus, to obtain (2S)-(+)-4 product when the lithium salt 2-Li is used in tetrahydrofuran (THF), the opposite enantiomer (2S)-(+)-6a is required; the (2S)-(+)-4 forms in 93.3% (Table 1, Batch E). In the case of the lithium salt 2-Li, the counter-ion (Li vs Na) or the solvent (THF vs DMF) or both may contribute to enhanced optical yields in comparison to the results when M = Na (Table 1, Batch A).

When (2R)-(-)-6b (i.e. glycidyl 3-nitrobenzene-sulfonate, the synthon containing a better leaving group). was reacted with the sodium salt 2-M (M = Na) in DMF the final product ((2S)-(+)-4) contained 94.1% of the (2S) enantiomer. (Table 1, Batch G). Utilizing the potassium salt 2-M (M = K) and (2R)-(-)-6b in DMF and subsequent reaction with the purine derivative (7) furnished (2S)-(+)-4 in even higher optical purity, containing 98.8% (Batch K) of the (2S)-(+) enantiomer. It should be noted that the optical yield improvement in changing from the sodium to potassium counter-ion is less dramatic using (2R)-(-)-6b, the compound containing the better leaving group. (Table 1, compare Batch A vs. Batch D and Batch G vs. Batch H).

The best optical yield has been obtained by using (2R)-(-)-6b and either the sodium salt 2-M (M = Na) or potassium salt 2-M (M = K) in THF. The (2S)-(+)-4 resulting from this reaction sequence contained 99.8% and 98.8% of the (2S) enantiomer, respectively. (Table 1, Batches I and K). Thus, by using the superior chiral synthon (2R)-(-)-6b in THF rather than DMF for the reaction with 2-M, either the potassium or the sodium counter-ions produce excellent results.

**TABLE 1**

**CHIRAL SYNTHESIS OF (2S)-(+)-4**

| Starting Materials | Solvent (1st Step) | (2S)-4 | Batch |
|---|---|---|---|
| (2R)-(-)-6a, 2-Na | DMF | 83.0% | A |
| (2R)-(-)-6a, 2-K | DMF | 97.2% | D |
| (2R)-(-)-6b, 2-Na | DMF | 94.1% | G |
| (2R)-(-)-6b, 2-K | DMF | 97.6% | H |
| (2R)-(-)-6b, 2-Na | THF | 99.8% | I |
| (2R)-(-)-6b, 2-K | THF | 98.8% | K |
| (2S)-(+)-6a, 2-Li | THF | 93.3% | E |

FIGURE 1

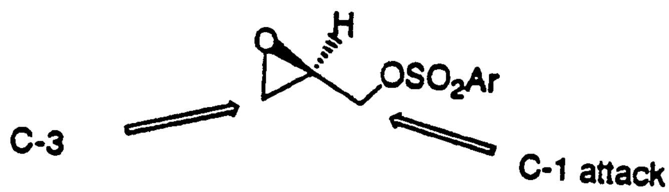


TABLE 2

CHIRAL SYNTHESIS OF (2R)-(-)-5

| Starting Material | M | (2S)-4 | (2R)-4 | Mechanism |
|---|---|---|---|---|
| (2S)-(+)-6a | Na | 17.0% | 83.0% | C-1 attack |
| (2R)-(-)-6b | Li | 8.0% | 92.0% | C-3 attack |

In summary, the present invention demonstrates chiral synthesis using derivatives of glycidol is much more complex than is stated in the current literature. In particular, in the examples cited herein, use of THF rather than DMF is critical to the formation of useful product with sufficient optical purity for biological utility. Secondly, the nature of the counter-ion plays a dramatic role both in the course of the nucleophilic reaction of glycidol derivatives as well as in the optical yield of the process. Finally, while current art suggests reaction with glycidol derivatives is limited to oxygen or carbon nucleophiles in order to achieve good optical yields, the present invention demonstrates that nitrogen nucleophiles may be used to give excellent yields of optically pure compounds for biological purposes.

Pharmaceutical compositions containing a compound prepared using the process of the present invention as the active ingredient in intimate admixture with a pharmaceutical carrier can be prepared according to conventional pharmaceutical compounding techniques. The carrier may take a wide variety of forms depending on the form of preparation desired for administration, e.g., intravenous, oral or parenteral. The composition may also be administered by means of an aerosol. In preparing the compositions in oral dosage form, any of the usual pharmaceutical media may be employed, such as, for example, water, glycols, oils, alcohols, flavoring agents, preservatives, coloring agents and the like in the case of oral liquid preparations (such as, for example, suspensions, elixirs and solutions); or carriers such as starches, sugars, diluents, granulating agents, lubricants, binders, disintegrating agents and the like in the case of oral solid preparations (such as, for example, powders, capsules and tablets). Because of their ease in administration, tablets and capsules represent the most advantageous oral dosage unit form, in which case solid pharmaceutical carriers are obviously employed. If desired, tablets may be sugar-coated or enteric-coated by standard techniques. for parenterals, the carrier will usually comprise sterile water, though other ingredients, for example, to aid solubility or for preservative purposes, may be included; injectable suspensions may also be prepared, in which case appropriate liquid carriers, suspending agents and the like may be employed. The pharmaceutical compositions will generally contain a dosage unit, e.g., tablet, capsule, powder, injection, teaspoonful and the like, from about 0.01 to about 50 mg/kg, and preferably from about 0.1 to about 10 mg/kg of the active ingredient.

The following examples describe the invention in greater particularity and are intended to be a way of illustrating but not limiting the invention. Some of the compounds in the examples were obtained as the hydrate. The water can be removed from the hydrates by drying at temperatures below the melting point of the compound.

Example 1

Synthesis of (2S)-(+)-6-[1-(1-(Bis-4-fluorophenyl)methyl)piperazin-4-yl)-2-hydroxy-3-propanylthio]purine

To a 500 mL, 3-neck flask equipped with a magnetic stir bar, nitrogen inlet, thermometer and addition funnel was added 50% sodium hydride (1.63 g, 0.034 mole) under a stream of nitrogen. The oil was removed by two washes with hexane (20 mL each). To the flask was added DMF (150 mL) and the suspension was cooled with stirring to 5°C. To the flask was then added 6-mercaptopurine hydrate (5.79 g, 0.034 mole) in one portion. Following hydrogen evolution the solution of the mercaptide anion was stirred at 5°C for 20 minutes and then warmed to room temperature. To this was added, with stirring over a one hour period, a solution of (2S)-(-)-(1,2-epoxypropyl)-4-[bis(4-fluorophenyl)methyl]piperazine (7.9 g, 0.023 mole) in DMF (150 mL). The reaction mixture was stirred at room temperature for 72 hours and then quenched by the addition of acetic acid (2.04 g, 0.034 mole). An insoluble precipitate was removed by filtration through Celite®. The filtrate was evaporated at 60°C (1 mm) to remove the DMF and the residue taken up in 10% methanol/methylene chloride (300 mL). The insoluble material formed was removed by filtration through Celite® and the solvent evaporated from the filtrate. The residue was taken up in methylene chloride (300 mL) and the insoluble material was again removed by filtration through Celite®. The filtrate was evaporated to a yellow foam which was chromatographed on 300 g of silica gel 60 (230-400 mesh) using 5% methanol/methylene chloride. Removal of solvent yielded 6.4 g (55%) of the title compound as a white foam after drying at 60°C (1 mm):mp 117-122°C; IR (KBr) 1602, 1568, 1506 $cm^{-1}$; UV λ max (ethanol) 331 nm (ε 870), 291 nm (ε 14800); MS (chemical ionization) 497 ($MH^+$); $[\alpha]^{22}_D$+8.7° (1% in ethanol); chiral HPLC, 99.8% (2S)-isomer; $^1H$ NMR (DMSO-$d_6$)δ8.63 (s, 1h), 8.42 (s, 1H), 7.42 (m, 4H), 7.11 (m, 4H), 4.35 (s, 1H), 3.91 (m, 1H), 3.66 (m, 1H), 3.30 (m,1H), 2.1-2.7 (m, 10H).

| | |
|---|---|
| Anal. Calcd for $C_{25}H_{26}F_2N_6OS \cdot \frac{1}{4} H_2O$: | |
| | C,59.39; H,5.38; N,16.62. |
| Found: | C,59.73; H,5.30; N,16.47. |

Example 2

Synthesis of (2R)-(-)-6-[1-(1-(Bis-(4-fluorophenyl)methyl)piperazin-4-yl)-2-hydroxy-3-propanylthio]purine

To a 500 mL, 3-neck flask equipped with a magnetic stir bar, nitrogen inlet, thermometer and addition funnel was added 50% sodium hydride (2.26 g, 0.047 mole) under a stream of nitrogen. The oil was removed by two washes with hexane (20 mL each). To the flask was added 6-mercaptopurine hydrate (8.0 g, 0.047 mole) in one portion. Following hydrogen evolution, the solution of the mercaptide anion was stirred at 5°C for 20 minutes and warmed to room temperature. To this was added, with stirring over a one hour period, a solution of (2R)-(+)-(1,2-epoxypropyl)-4-[bis(4-fluorophenyl)-methyl]-piperazine (10.8 g, 0.031 mole) in DMF (200 mL). The reaction mixture was stirred at room temperature for 72 hours and then quenched by the addition of acetic acid (2.82 g, 0.047 mole). The insoluble precipitate was removed by filtration through Celite®. The filtrate as evaporated at 60°C (1 mm) to remove DMF and the residue taken up in 10% methanol/methylene chloride (400 mL). The precipitate as removed by filtration through Celite® and the solvent evaporated. The residue was taken up in methylene chloride (400 mL), the insoluble material again removed by filtration through Celite® and the filtrate was evaporated to a yellow foam. Chromatography of this residue through 400 g of silica gel 60 (230-400 mesh) using 5% methanol/methylene chloride and removal of solvent yielded 11.2 g (72%) of the title compound as a white foam after drying at 60°C (1 mm):mp 117-125°C; IR (KBr) 1602, 1568, 1506 $cm^{-1}$; UV λ max (ethanol) 331 nm (ε 759), 291 nm (ε 16700); MS (chemical ionization) 497 ($MH^+$); $[\alpha]^{22}_D$-9.0° (1% in ethanol); $^1H$ NMR (DMSO-$D_6$) δ 8.65 (s, 1 H), 8.45 (s, 1 H), 7.45 (m, 4 H), 7.12 (m, 4 H), 4.35 (s, 1 H), 3.94 (m, 1 H), 3.69 (m, 1 H), 3.30 (m, 1 H), 2.1-2.7 (m, 10 H).

| | |
|---|---|
| Anal. Calcd for $C_{25}H_{26}F_2N_6OS \cdot \frac{1}{4} H_2O$: | |
| | C, 59.39; H, 5.38; N, 16.62. |
| Found: | C, 59.58; H, 5.20; N, 16.93. |

Example 3

Synthesis of (2R)-(+)-(1,2-Epoxypropyl)-4-[bis(4-fluorophenyl)methyl]piperazine

To a 1 L, 3-neck flask equipped with a mechanical stirrer, nitrogen inlet, thermometer and addition funnel was added 50% sodium hydride (2.64 g, 0.055 mole) under a stream of nitrogen. The oil was removed by two washes with hexane (25 mL each). To the flask was then added THF (250 mL) and 4,4′-difluorobenzhydrylpiperazine (14.42 g, 0.05 mole). To this mixture was added with stirring at 20-25°C a solution of previously recrystallized(2S)-(+)-glycidyl 3-nitrobenzenesulfonate (12.96 g, 0.05 mole). The reaction mixture was stirred at room temperature for 24 hours and another portion of 4,4′-difluorobenzhydrylpiperazine (1.0 g, 0.0035 mole) was added.

The mixture was stirred for an additional 24 hours and filtered through Celite® to remove insoluble material. After solvent evaporation, the crude residue was purified by chromatography on 1 kg of silica gel 60 (230-400 mesh) using 10% acetone/methylene chloride. The yield of the purified product obtained as a yellow oil after solvent removal at 60°C (1 mm) was 13.4 g (78%): IR (neat) 2812, 1603, 1506 $cm^{-1}$; UV λ max (ethanol) 274 nm (∊ 1129), 267 nm (∊ 1430), 222 nm (∊ 9355); MS (chemical ionization ) 345 ($MH^+$); $[\alpha]^{22}_D$ + 12.3° (1% in ethanol); chiral HPLC, 99.9% (2R)-isomer; $^1H$ NMR ($CDCl_3$) δ 7.33 (m, 4 H), 6.95 (m, 4 H), 4.23 (s, 1 H). 3.07 (m, 1 H), 2.2-2.75 (m, 12 H).

Anal. Calcd for $C_{20}H_{22}F_2N_2O$: C, 69.75; H, 6.44; N, 8.13.
Found: C, 69.52; H, 6.64; N, 8.04.

Example 4

Synthesis of (2s)-(-)-(1,2-Epoxypropyl)-4-[bis 4-fluorophenyl)methyl]piperazine

To a 1 L, 3-neck flask equipped with a mechanical stirrer, nitrogen inlet, thermometer and addition funnel was added 50% sodium hydride (1.78 g, 0.037 mole) under a stream of nitrogen. The oil was removed by two washes with hexane (25 mL each). To the flask was then added THF (150 mL) and 4,4′-difluorobenzhydrylpiperazine (9.75 g, 0.034 mole). To this mixture was added with stirring at 20-25°C over a 2 hour period a solution of previously recrystallized (2R)-(-)-glycidyl 3-nitrobenzenesulfonate (8.75 g, 0.034 mole) dissolved in THF (200 mL). The reaction mixture was stirred at room temperature for 20 hours and filtered through Celite® to remove precipitate. After solvent evaporation, the crude residue was purified by chromatography on 300 g of silica gel 60 (230-400 mesh) using 10% acetone/methylene chloride. The yield of the purified product obtained as a yellow oil after solvent removal at 60°C (1 mm) was 8.0 g (68%): $[\alpha]^{22}_D$-11.9° (1% in ethanol); chiral HPLC, 99.75% (2S)-isomer; $^1H$ NMR ($CDCl_3$) δ 7.33 (m, 4 H), 6.95 (m, 4 H), 4.23 (s, 1 H), 3.07 (m, 1 H), 2.2-2.75 (m, 12 H).

## Claims

1. A process for preparing optically active isomers of compounds of the formula

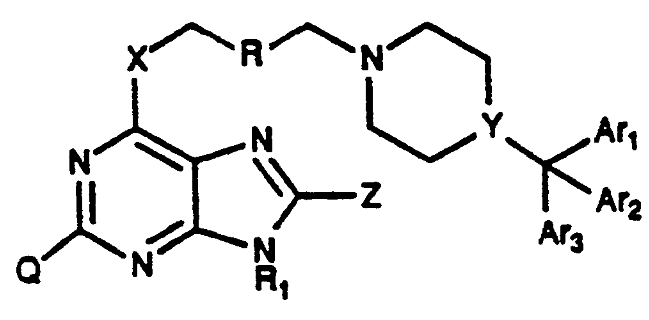

wherein

R is

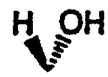

or

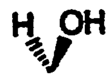

;

$R_1$ is hydrogen, $C_1$-$C_4$ alkyl, cycloalkyl, benzyl, $C_1$-$C_4$ alkenyl, $C_1$-$C_4$ alkynyl, epoxypropyl or cyclic ether;

X is S, O, NH, $NR_2$ wherein $R_2$ is $C_1$-$C_4$ alkyl;

Y is N or C =;

$Ar_1$, $Ar_2$ and $Ar_3$ are independently hydrogen, $C_1$-$C_4$ alkyl phenyl, substituted phenyl wherein the substituent is $C_1$-$C_4$ alkyl, $C_1$-$C_4$ alkoxy, trifluoromethyl, halo such as fluoro, chloro, bromo, iodo or perhalo, naphthyl, pyridyl or thienyl, wherein at least one of $Ar_1$, $Ar_2$ or $Ar_3$ is aromatic;

Z is hydrogen, CN or $CO_2R_3$ wherein $R_3$ is hydrogen $C_1$-$C_4$ alkyl, hydroxy or halogen such as fluoro, chloro or bromo; and

Q is hydrogen, halogen, amino, $C_1$-$C_4$ alkyl or hydroxy;

with the proviso that when Y is C =, $Ar_3$ is not present,

comprising the steps of:

a) reacting an optically active glycidyl derivative of the formula

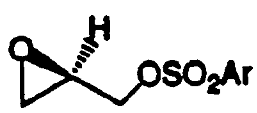

or

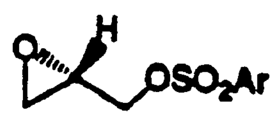

wherein Ar is a 4-methylphenyl group or a 3-nitrophenyl group with a piperazine derivative salt of the formula

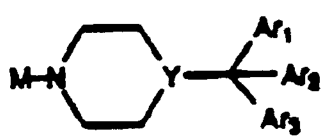

wherein M is Na, K or Li and Y, $Ar_1$, $Ar_2$ and $Ar_3$ are as defined above in a solvent to produce an optically active epoxypiperazine derivative of the formula

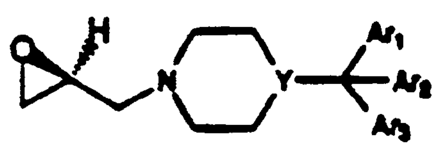

;

and

b) reacting the optically active epoxypiperazine derivative with a sodium salt of a purine derivative of the formula

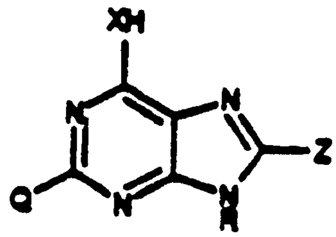

2. The process of claim 1 wherein the solvent is tetrahydrofuran or dimethylformamide.

3. The process of claim 1 wherein R is

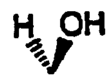

,

M is Na or K, and the glycidyl derivative is a (2R)-(-)-enatiomer.

4. The process of claim 3 wherein Ar is the 3-nitrophenyl group and the solvent is tetrahydrofuran.

5. The process of claim 1 wherein R is

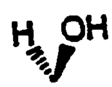

,

M is Li, and the glycidyl derivative is a (2S)-(+)-enantiomer.

6. The process of claim 5 wherein the solvent is tetrahydrofuran.

7. The compound (2S)-(+)-6-[1-(1-(Bis(4-fluorophenyl)methyl)piperazin-4-yl)-2-hydroxy-3-propanylthio]purine.

8. The compound (2R)-(-)-6-[1-(1-(Bis-(4-flurophenyl)methyl)piperazin-4-yl)-2-hydroxy-3-propanylthio]purine.

9. The compound (2R)-(+)-(1,2-Epoxypropyl)-4-[bis(4-fluorophenyl)-methyl]piperazine.

10. The compound (2S)-(-)-(1,2-Epoxypropyl)-4-[bis(4-fluorophenyl)-methyl]piperazine.

European Patent Office

# EUROPEAN SEARCH REPORT

Application Number

EP 91 30 3564

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A<br><br>D | EP-A-0331511 (ORTHO PHARMACEUTICAL CORP.)<br>* claims 1, 10, 15 *<br>& US-A-4876257<br>--- | 1, 7-10 | C07D473/38<br>C07D473/00 |
| A,D | JOURNAL OF ORGANIC CHEMISTRY, vol. 51, 1986, pages 3710 - 3712; J.M. KLUNDER et al.: "Asymmetric Epoxidation of Allyl Alcohol: Efficient Routes to homochiral β-Adrenergic Blocking Agents"<br>--- | 1 | |
| A,D | JOURNAL OF ORGANIC CHEMISTRY, vol. 54, 1989, pages 1295 - 1304; J.M. KLUNDER et al.: "Arenesulfonate Derivatives of Homochiral Glycidol: Versatile Chiral Building Blocks for Organic Synthesis"<br>----- | 1 | |
| | | | TECHNICAL FIELDS SEARCHED (Int. Cl.5) |
| | | | C07D473/00 |

The present search report has been drawn up for all claims

| Place of search | Date of completion of the search | Examiner |
|---|---|---|
| BERLIN | 12 JULY 1991 | HASS C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)